Europäisches Patentamt

(19) European Patent Office

Office européen des brevets

(11) Publication number: **0 286 858**
**A1**

## EUROPEAN PATENT APPLICATION

(21) Application number: **88104198.2**

(22) Date of filing: **16.03.88**

(51) Int. Cl.⁴ **A61N 5/10 , G21K 1/02**

(30) Priority: **31.03.87 US 33134**

(43) Date of publication of application:
**19.10.88 Bulletin 88/42**

(84) Designated Contracting States:
**DE FR GB NL SE**

(71) Applicant: **Siemens Aktiengesellschaft Berlin und München**
**Wittelsbacherplatz 2**
**D-8000 München 2(DE)**

(72) Inventor: **Barthelmes, Norbert**
**Schobertweg 49**
**D-8520 Erlangen(DE)**

(54) **Collimator for a linear electron accelerator.**

(57) A collimator for defining a radiation field of a high energy beam (1) emitted by a source along a beam axis (4). The collimator comprises two pairs of jaws. One pair of jaws (13, 14) is disposed on top of the other pair (16, 17) and staggered by 90° against it so that a closed passageway for the beam is defined. Each jaw has an outer (18, 19, 21, 22) and an inner part (23, 24, 26). The inner part is inserted in the outer part and rotatable such that its inner surface, which defines one sidewall of the passageway, is tilted against the beam axis (4). The jaw is laterally shiftable, and both motions are coordinated so that when the jaw moves, its sidewall-defining surface is always aligned with the geometric divergency of the beam (1).

FIG 1

## BACKGROUND OF THE INVENTION

The invention relates to a collimator for high energy beams. In particular, it relates to a collimator for defining a treatment field for a linear electron accelerator (LINAC) used in radiotheraphy.

In a typical electron accelerator, as described for instance in U.S. Patent 4,343,997, four movable jaws define a closed passageway for the beam. Each of these block-like jaws is "focused", that is, its inner surface is aligned with the geometrical beam divergency and can be moved along a curved path so that the cross-section of the passageway may be adjusted while the inner jaw surface alignment remains undisturbed. To avoid radiation leaks between adjacent jaws, the system is divided into pairs of opposite jaws positioned on top of each other along the beam axis. Such a structure allows different field sizes and provides field boundaries which are blurred only because the source is usually not punctiform but extended. The collimator requires, however, sufficient clearance between the two jaw pairs; it is thus very extended along the beam axis, and the half shadows (= penumbras) defined by the different jaws pairs vary significantly from each other.

If the jaws are moved transverse to the beam axis, as described for instance in U.S. Patents 4,198,570 or 4,467,197, the overall length and the asymmetry in the field margin can be reduced. However, there is only one position in which the jaws are ideally focused.

To reduce the impact of the jaw position on the penumbra German Offenlegungsschrift 33 11 870 suggests to provide the transversely shiftable jaw with a convex beam defining surface so that the passageway for the beam is flared out. This way the beam is tangent to the curved surface and moves downstream when the jaw is shifted away from the beam axis. In practice, the field margin is still related to the diameter of the area; it becomes broader with decreasing field size, which is especially troublesome.

Canadian patent 576,973 teaches a collimator which is compact and produces field boundaries which are not only symmetrical but which are well-defined for a variety of different field sizes as well. In this structure, each block is replaced by a stack of laterally movable plates spaced apart from each other. The stacks are nested within each other to form the required passageway. To support the movable plates, their structure is interleaved with a similar structure with fixed bars. Each plate slidably on one of these bars, and all plates of opposite stacks are movable in a coordinated manner so that the length ratio of the treatment field may be varied, with the passageway kept focused. So far, this concept has not been commercialized, apparently because the moving means are relatively complicated and must overcome high frictional forces.

In all of the above-mentioned collimators, the penumbra is a function of the field size and cannot be varied. For specific applications, however, freely trimmable field margins would be desirable. A prominent example is a multi-field treatment, where several radiation fields are added to cover a very large target volume. Here, detrimental intensity fluctuations on the suture points between adjacent fields can best be curbed, if there is an overlap between neighboring fields having a proper intensity distribution in the joined region (see, for example, "Strahlentherapie", 156 (1980) p. 115 to 119).

It is an object of this invention to provide a collimator which is capable of producing fields with variable sizes and sharply focused boundaries.

It is another object of this invention to provide a collimator for creating an irradiation field whose margin has at least an approximately constant width around the perimeter of the treatment field.

It is a further object of this invention to provide a collimator which is compact.

It is still a further object of this invention to provide a collimator whose movable parts may be actuated with relatively small forces.

It is yet another object of this invention to provide a collimator with a simple mechanism for shifting its movable parts.

It is a further object of this invention to provide a collimator for generating treatment fields with variable sizes and adjustable margin zones.

It is still a further object of this invention to provide a collimator for confining rectangular treatment fields whose straight margin sections maybe be adjusted independently from each other.

It is yet another object of this invention to improve upon known collimators of this type.

## SUMMARY OF THE INVENTION

The invention is directed to a collimator for defining the radiation field of a high energy beam emitted by a source along a beam axis. The collimator has two pairs of jaws which lie in two different planes perpendicular to the beam axis. The jaws of each pair are opposite to each other across the beam axis, and the jaws of different pairs are 90° apart from each other with respect to the beam axis. Each jaw consists of an outer and an inner part. The inner part is rotatable around an axis orthogonal to the beam axis and has a plane surface forming a sidewall of a passageway for the high energy beam. Upon rotation of the inner part, the sidewall is tilted so that it becomes more or less divergent from the beam axis. The collimator is further provided with means for shifting each jaw towards and away from the beam axis and means for rotating the inner part of each jaw.

A collimator according to the invention can define an irradiation area having borderlines which are narrow at different sizes as well as in both main axes of the field. The arrangement is compact so that, if one substitutes a jaw system according to the invention for a conventional collimator with swinging jaws, more space between the beam source and the jaws is available and the quality of the beam boundary is improved.

According to one aspect of the invention, the shifting means are synchronized with the rotating means such that if the outer part is moved laterally, the associated inner part is automatically rotated into a position, where its sidewall-defining surface is focused. Preferably, the outer part is driven by a spindle mechanism, while the inner part is pivoted in its rotational axis by arms mounted on the associated outer part. It is rotated by a lever having its one end eccentrically fixed to the inner part and its other end guided along a straight line.

In case the shifting means and rotating means for each jaw are decouples, the penumbra may be varied at different field sizes. If further the shifting means for different outer parts as well as the rotating means for different inner parts operate independently, different field formats with individual margins along different field edges may be generated.

In a preferred embodiment, the inner part is a partial cylinder, i.e. a cylinder lacking a segment cut away along a chordal plane; this tangential surface defines the sidewall. The partial cylinder is inserted in a matched receptacle of the associated outer part. This way both parts form a solid plate-like block whose absorption characteristics for the high energy beam is independent of the jaw position.

## BRIEF DESCRIPTION OF THE DRAWING

Fig. 1 shows in a schematic sideview the beam bending and defining part of a LINAC containing a first embodiment of the invention;

Fig. 2 is a sideview of the embodiment of Fig. 1, shown in more detail;

Fig. 3 shows from the embodiment of Fig. 1 another sideview, the line of sight being marked by an arrow in Fig. 2; and

Fig. 4 shows from another embodiment one jaw, in a sideview.

## DETAILED DESCRIPTION OF PREFERRED EMBODIMENTS

For the sake of clarity, some parts of the jaw system have been omitted. Throughout the drawings, corresponding elements are referred to by like numerals.

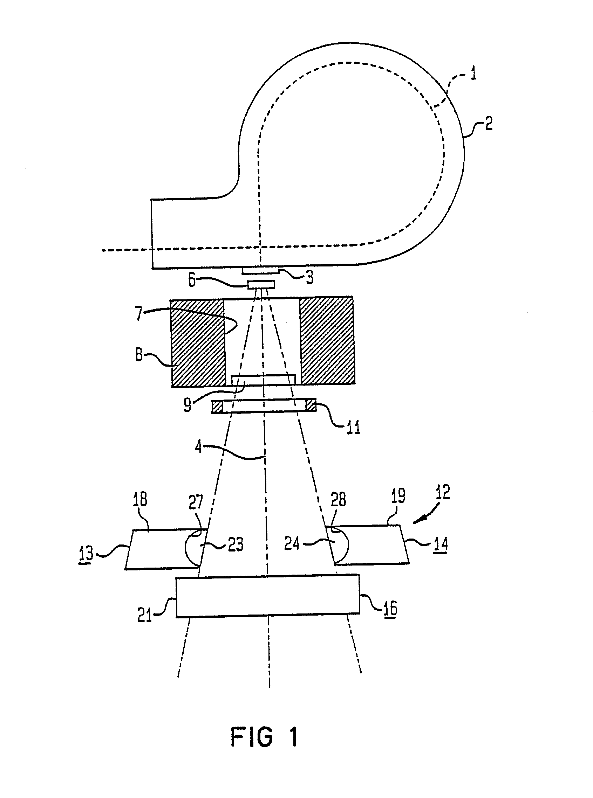

Fig. 1 shows from a LINAC which can be operated in an electron mode, the beam bending and defining system.

An electron beam 1 leaving an accelerating wave guide (not shown) is diverted into a circular path in a bending magnet 2 and emitted through a window 3 along an axis 4. The beam then strikes a first scattering foil 6, passes through a passageway 7 defined by a shielding block 8, and hits a second scattering foil 9. Thereafter, it is sent through an electron dosage member 11 and finally through a jaw system 12.

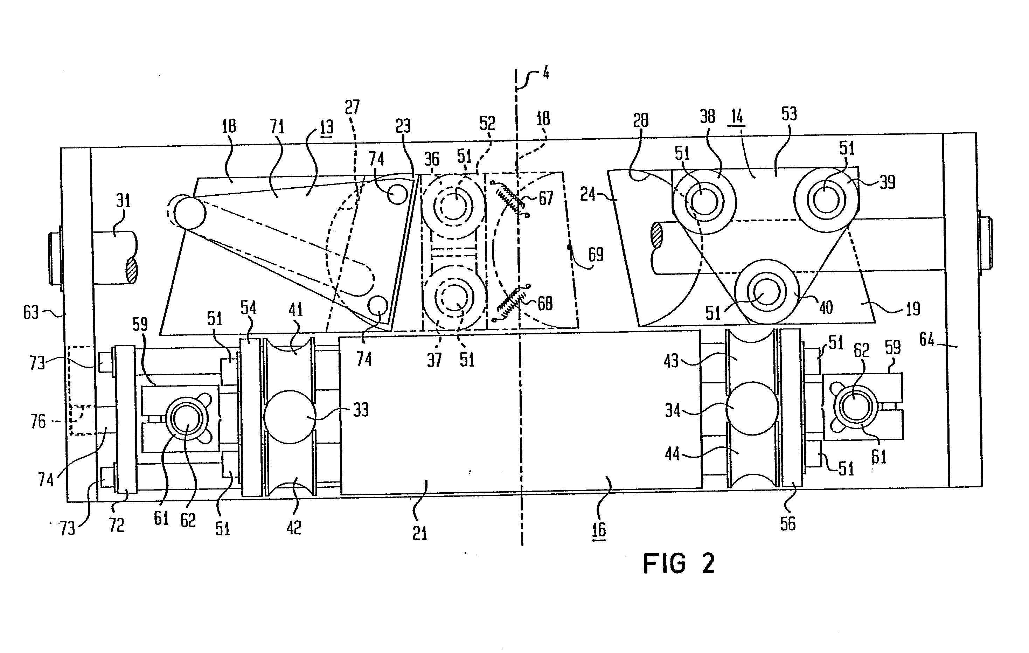

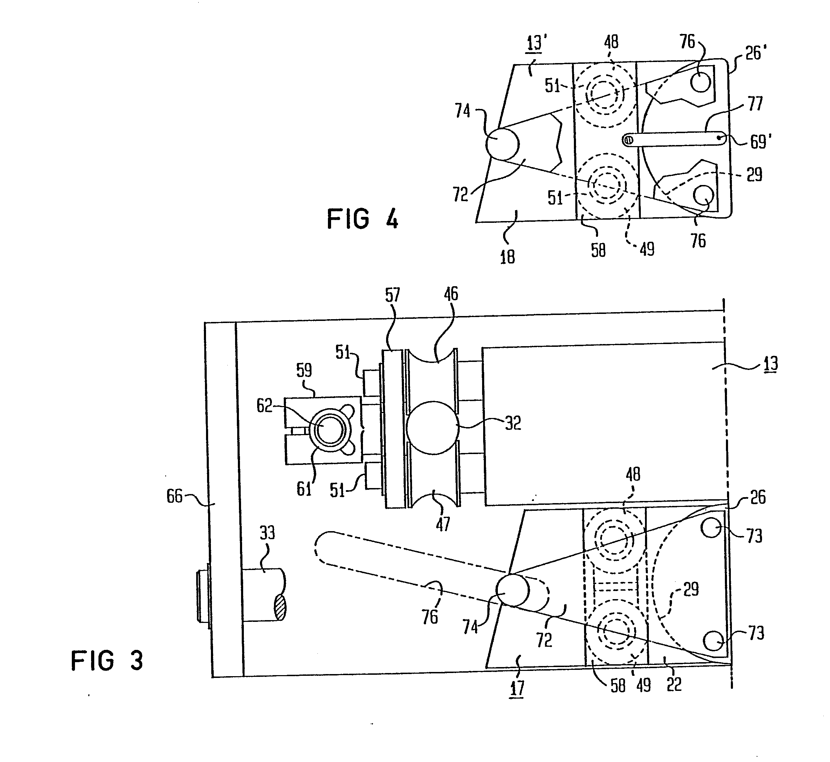

This system, which is depicted in Figs. 2 and 3 in more detail, contains four jaws which are grouped in two pairs around the beam axis 4. The jaws of the first pair, referred to by numerals 13, 14, extend in a first plane perpendicular to the beam axis on opposite sides thereof. The jaws of the second pair, designated with numerals 16, 17 are arranged in a second plane perpendicular to axis 4 and again opposite to each other with respect to this axis; however, jaws of different pairs are staggered by 90° with respect to the beam axis 4. Each jaw is composed of two parts, an outer part 18, 19, 21, 22 and an inner part 23, 24, 26. The inner part is shaped as a semi-cylinder provided with a plane surface 25 and received in a matching recess 27, 28, 29 of the outer part. The diameter of the semi-cylinder corresponds to the width of the outer part so that both parts form a plate without any cavities which could weaken the beam characteristics of the jaw.

Each jaw is disposed between two guiding rods 31, 32, 33, 34 which are parallel to each other and perpendicular to the beam axis 4. The arrangement is such that the jaws of one pair share a pair of parallel guiding rods. At its sides adjacent the guiding rods each outer part has a set of rolls 36, 37; 38 39, 40; 41, 42; 43, 44; 46, 47; 48, 49. The rolls of each set encompass a guiding rod so that each jaw can glide along a rod axis. Each jaw has, as can be seen from Fig. 2 and 3, a pair of rolls at one side and a roll triplet at the other side. Such an arrangement allows relatively high tolerances in the position and orientation of the rolls as well as the rods.

Each roll is fixed to its outer part by means of a bolt 51, and the bolts of each set of rolls are in turn kept in place by a plate 52, 53, 54, 56, 57, 58. This plate serves also to carry a block-like clamp 59. This clamp contains a nut 61 which is threaded on a spindle 62, thus providing a drive mechanism. If the spindle is rotated, the jaw moves along its associated guiding rods. Rods and spindles are secured in sidewalls 63, 64, 66 of a frame surrounding the jaw system.

The inner part of each jaw is rotatably attached to its associated outer part by springs from which only springs 67, 68 at one side of outer part 18 are depicted. Each semi-cylinder can be turned around an axis through its center of curvature which is referred to by numeral 69 for outer part 18. To this end, a lever formed as a three-cornered plate 71, 72 is attached-via two bolts 73 each extending through a corner of plate 71, 72 - ton one of the front sides of the semi-cylinder. The third corner of plate 71, 72 is provided with a pin 74 which extends parallel to the rotational axis of its associated inner part and glides in an elongated straight slot 76 extending transversely to the same axis. This slot, which is placed in the adjacent lateral framewall, is tilted against the beam axis 12 so that when the jaw is moved away from axis 4 its sidewall-defining surface remains adjusted to the beam cone. This means that if the beam is emitted by a point source, the plane defined by surface 25 intersects axis 4 at the source site. In case the source is extended, the plane is tangential to it.

Fig. 4 shows from another embodiment one jaw 13' in a side view. This jaw differs from the jaws of the previous figures in that its inner part 26' has a slightly modified shape and is fixed differently to its outer part 18. As can be seen from Fig. 4, the cross-section of inner part 26' is again a part of a circular area, the missing segment is, however, smaller than the remaining area. Furthermore, inner part 26' is fastened to outer part 18 by angular arms (arm 77 is shown) rather than springs. At each side adjacent to the guiding rod, there is provided one arm mounted at one of its ends on outer part 18 and projecting with its other end which is hooked, into a hole of inner part 26' coaxial with rotational axis 69'. This suspension mechanism, which also affords focused jaws, is especially fail safe and therefore preferable in medical applications.

Having thus described the invention with particular reference to preferred forms thereof, it will be obvious to those skilled in the art to which the invention pertains, after understanding the invention, that various changes and modifications may be made therein without departing from the spirit and scope of the invention as defined by the claims appended hereto. So, the collimator may confine other high energy beams rather than e-beams, e.g. x-ray radiation. Furthermore, it is not always necessary to provide all jaws of the collimator with tiltable inner surfaces.

## Claims

1. A collimator for adjusting the shape of an area irradiated by a high energy beam which is emitted along a beam axis, comprising:
   (a) a first pair of jaws disposed on opposite sides of the beam axis in a first plane substantially perpendicular to the beam axis;
   (b) a second pair of jaws disposed on opposite sides of the beam axis in a second plane substantially perpendicular to the beam axis, wherein each jaw comprises (c1) an outer part and (c2) an inner part attached to the outer part rotably around an axis spaced from and normal to the beam axis and having a plane surface which forms a predetermined angle with the beam axis and defines a sidewall of a passageway for the high energy beam;

   (d) means for shifting each jaw transversely to the beam axis; and
   (e) means for rotating the inner part of each jaw and thereby varying the predetermined angle.

2. A collimator according to claim 1, wherein the shifting means is synchronized with the rotating means so that each sidewall defines a plane which in all transverse jaw positions intersects the beam axis at a substantially identical location.

3. A collimator according to claim 1, wherein the inner part of each jaw is shaped as a partial cylinder with a chordal plane defining said sidewall.

4. A collimator according to claim 3, wherein the partial cylinder is a semi-cylinder.

5. A collimator according to claim 1, wherein the inner part is inserted in a recess of the associated outer part.

6. A collimator according to claim 5, wherein the recess of the outer part has a shape which mates with the inserted inner part.

7. A collimator according to claim 6, wherein the inner and outer parts of each jaw form a plate-like solid block.

8. A collimator according to claim 3, wherein the curved perimeter of the partial cylinder covers more than a half circle.

9. A collimator according to claim 8, wherein the inner part is attached to the associated outer part by arms having two ends each arm being mounted at its one end on the associated outer part and projecting with its other end into a hole of the inner part coaxial to its axis of rotation.

10. A collimator according to claim 1, wherein the rotating means comprises a lever with two ends, one end of the lever being eccentrically attached to the inner part and the other end of the lever being guided along a predetermined line.

11. A collimator according to claim 10, further comprising a frame with lateral walls surrounding the jaw pairs, each lateral wall having a slot for receiving the other end of the lever.

12. A collimator according to claim 1, wherein the shifting means comprise two pairs of parallel guiding rods and wherein each jaw is suspended between two guiding rods, opposite jaws sharing the guiding rods of guiding rod pairs.

13. A collimator according to claim 1, wherein the shifting means comprise a spindle drive for each jaw.

14. A collimator for adjusting the shape of an area irradiated by a high energy beam which is emitted along a beam axis, comprising:

(a) a first pair of jaws disposed on opposite sides of the beam axis in a first plane substantially perpendicular to the beam axis;

(b) a second pair of jaws disposed on opposite sides of the beam axis in a second plane substantially perpendicular to the beam axis, wherein at least one of the jaws comprises (c1) an outer part and

(c2) an inner part attached to the outer part rotably around an axis spaced from and normal to the beam axis and having a plane surface which forms a predetermined angle with the beam axis and defines a sidewall of a passageway for the high energy beam;

(d) means for shifting each jaw towards and away from the beam axis; and

(e) means for rotating said inner part of said jaw and thereby varying the predetermined angle.

**FIG 1**

FIG 2

0 286 858

FIG 4

FIG 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A-2 519 465 (CGR-MEV) <br> * Whole document * <br> ----- | 1-10 | A 61 N 5/10 <br> G 21 K 1/02 |

**TECHNICAL FIELDS SEARCHED (Int. Cl.4)**

A 61 N
A 61 B
G 21 K

The present search report has been drawn up for all claims

| Place of search | Date of completion of the search | Examiner |
|---|---|---|
| THE HAGUE | 22-07-1988 | LEMERCIER D.L.L. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)